# EUROPEAN PATENT SPECIFICATION

(11) **EP 4 326 339 B1**
(45) Date of publication and mention of the grant of the patent: **02.04.2025**
(21) Application number: 22734477.7
(22) Date of filing: 20.04.2022
(51) Int. Cl.: A61K 48/00, C12N 15/86, C12N 15/864, A61P 27/02, C07K 14/435

(54) **COMPOSITIONS AND METHODS FOR TREATING RETINAL DEGENERATIVE DISORDERS**
ZUSAMMENSETZUNGEN UND VERFAHREN ZUR BEHANDLUNG VON DEGENERATIVEN NETZHAUTERKRANKUNGEN
COMPOSITIONS ET MÉTHODES DE TRAITEMENT DE TROUBLES DÉGÉNÉRATIFS DE LA RÉTINE

(30) Priority: 20.04.2021 EP 21169473
(43) Date of publication of application: 28.02.2024
(73) Proprietor: Institut National de la Santé et de la Recherche Médicale (INSERM), 75013 Paris (FR); Sparingvision, 75008 Paris (FR); Centre National de la Recherche Scientifique (CNRS), 75016 Paris (FR); SORBONNE UNIVERSITE, 75006 Paris (FR)
(72) Inventor: DALKARA, Deniz, 75013 PARIS (FR); KHABOU, Hanen, 93160 NOISY LE GRAND (FR); LORGET, Florence, San Francisco, CA 94116 (US); SAHEL, José, Pittsburgh, PA 15213 (US); CARDILLIA-JOE, Simon, 69100 VILLEURBANNE (FR)
(74) Representative: Plasseraud IP
(86) International application number: PCT/EP2022/060462
(87) International publication number: WO 2022/223644

(56) References cited:
- EP-A1- 3 892 738
- WO-A1-2016/185037
- WO-A1-2017/120294
- WO-A1-2019/094904
- WO-A1-2019/122403
- WO-A1-2021/204407
- SIMON C J ET AL: "Gene therapy to increase and maintain light sensitivity in cones during retinal degeneration", HUMAN GENE THERAPY, vol. 30, no. 11, 1 November 2019 (2019-11-01), pages A111, XP055847462
- MARRON FERNANDEZ DE VELASCO EZEQUIEL ET AL: "GIRK2 splice variants and neuronal G protein-gated K+ channels: implications for channel function and behavior", vol. 7, no. 1, 1 December 2017 (2017-12-01), pages 1639, XP055848160, Retrieved from the Internet <URL:https://www.ncbi.nlm.nih.gov/pmc/articles/PMC5431628/pdf/41598_2017_Article_1820.pdf> DOI: 10.1038/s41598-017-01820-2

## Description

### Field of the invention

The present invention relates to the treatment of retinal neurodegenerative disorders, and more particularly to the treatment by maintaining the integrity of the cone photoreceptors and by reactivating cones which have already lost their outer segment.

### Background Art

Neurodegenerative disorder encompasses a range of seriously debilitating conditions that are characterized by neuron degeneration.

Photoreceptors, such as rods and cones, are light-sensitive sensory neurons found on the posterior layer of the retina. They are also called photoreceptor cells or photoreceptor neurons.

Retinal neurodegenerative disorders or retinal degenerative diseases encompass different subgroups of pathologies: Rod-cone dystrophies, Cone dystrophies, Cone-rod dystrophies, and atrophic age-related macular degeneration.

Rod-cone dystrophies (RCD), such as retinitis pigmentosa (RP), are genetically heterogeneous retinal neurodegenerative diseases characterized by the progressive death of rod photoreceptors followed by the consecutive loss of cones. RP is one of the most common forms of inherited retinal degeneration, affecting around 1:3,500 people worldwide (1), which represents 2 million patients worldwide. Mutations causing RP in over 63 distinct genes have been identified to date with a significant proportion of these mutations in rod-specific transcripts.

Cones dystrophies are characterized by the vision loss (age of onset ranging from the late teens to the sixties), sensitivity to bright lights, and poor color vision. Therefore, patients see better at dusk. Visual acuity usually deteriorates gradually, but it can deteriorate rapidly to 20/200. Later, in more severe cases, it drops to "counting fingers" vision. Color vision testing using color test plates (HRR series) reveals many errors on both red-green and blue-yellow plates.

Cone-Rod Dystrophies (CRD) refer to a group of inherited retinal degenerations (1:30 - 40,000 people) that affect the photoreceptor (light sensing) cells that are responsible for capturing images from the visual field. These cells line the back of the eye in the region known as the retina. Cone photoreceptor cells are present throughout the retina, but are concentrated in the central region (the macula). They are useful for central (reading) vision. Rod photoreceptor cells are present throughout the retina except for the very center of the macula called the fovea where only cones are present. They are responsible for night vision.

In contrast to typical retinitis pigmentosa (known as the Rod-Cone Dystrophies), which results from the loss of rod cells and later the cone cells, Cone-Rod Dystrophies can reflect the opposite sequence of events, where cone cells are primarily first affected with later loss of rods. The degree of vision loss becomes more severe over time. There are multiple types of Cone-Rod Dystrophies, which are determined by their genetic cause and pattern of inheritance.

Atrophic age-related macular degeneration (AMD) or advanced dry AMD, is an advanced form of age-related macular degeneration that can result in the progressive and irreversible loss of retina (photoreceptors, retinal pigment epithelium, choriocapillaris) which can lead to a loss of visual function over time (22, 23, 24, 25). It is estimated that atrophic AMD affects more than 5 million people worldwide and approximately 1 million patients in the US (26, 27), which is similar to the prevalence of neovascular (wet) AMD, the other advanced form of the disease.

In patients suffering from RCD, in particular from RP, vision loss develops in three successive steps.

RP patients initially present with loss of vision under dim-light conditions (night vision loss), corresponding to the loss of function and degeneration of rods with relative preservation of macular cone-mediated vision. This is felt as a minor handicap, where patients retain an almost normal way of life (19).

The disease then progresses through a second more incapacitating step resulting from the loss of function and degeneration of cones. In this later stage of the degeneration, cones degenerate in the periphery leading to the well-known tunnel vision. Indeed, the center of the retina represents 5% of all photoreceptors in human and most mammals, and when this part is conserved, patients maintain a high acuity but restricted field of vision (20).

The final stage of the disease corresponds to the degeneration of the foveolar cone photoreceptors, which leads to a total loss of vision in the patient. In this stage, some of the cones present viable cell bodies despite the degeneration of their light sensing outer segments.

In modern society, in which much of the environment is artificially lit, and many activities rely on high acuity color vision, thus the retention of cone-mediated sight in RP patients would lead to a significant improvement in quality of life.

The loss of cones in RP subsets caused by rod-specific mutations is not perfectly understood, although several mechanisms, which are not necessarily mutually exclusive, have been proposed. Some hypothesized mechanisms implicate a 'bystander effect' whereby cone death is a consequence of the release of toxic byproducts from the degeneration of surrounding rods, or as a result of the loss of contact with rods, retinal pigment epithelium (RPE) or Müller glia. Alternatively, activation of Müller cells and the release of toxic molecules may play a role. Another hypothesis is that the quantities of oxygen or retinoids delivered to the photoreceptor layer by the RPE from the choroidal blood circulation are excessive and toxic as the metabolic load of rods is lost (2). Punzo et al. showed evidence that in murine models of retinal degeneration cones die in part as a result of starvation and nutritional imbalance, driven by the insulin/mammalian target of rapamycin pathway (3). Additionally, it has been suggested that the loss of a survival factor secreted by rods and required for cone survival may contribute to cone loss (4, 5).

In agreement with the last hypothesis, transplanted healthy retinal tissue has been shown to support cone survival in areas distant from the grafted tissue in the rd1 mouse (6, 7).

International patent application WO2008/148860A1 describes a family of trophic factors, called rod-derived cone viability factor (RdCVF) and RdCVF2 that are able to increase neuron survival and are useful for treating and/or preventing neurodegenerative disorders such as RP.

The rod-derived cone viability factor (RdCVF) was originally identified from a high-throughput method of screening cDNA libraries as a candidate molecule responsible for this rescue effect (4). Rods secrete RdCVF, and therefore, as rods die, the source of this paracrine factor is lost and RdCVF levels decrease. The loss of expression of RdCVF, and secreted factors like it, may therefore contribute to the secondary wave of cone degeneration observed in rod-cone dystrophies.

RdCVF has been shown to mediate cone survival both in culture (8) and when injected subretinally in mouse and rat models of recessive and dominant forms of retinitis pigmentosa (4, 9). In 2010, Léveillard and Sahel (21) have shown that expression of RdCVF preserves cone-mediated vision by allowing the maintenance of cone outer segments, thereby increasing cone functional life.

Besides, it has been shown that disruption of Nxnl1, the gene encoding RdCVF, renders mouse photoreceptors increasingly susceptible to photoreceptor dysfunction and cone loss over time (10).

Nxnl1 encodes two protein isoforms through differential splicing. The isoform mediating cone survival, RdCVF is a truncated thioredoxin-fold protein of its longer counterpart, RdCVFL, which includes a C-terminal extension conferring enzymatic thioloxidoreductase activity (11). RdCVFL, which contains all the amino acids of RdCVF, is encoded by exons 1 and 2 of the Nxnl1 gene and is a member of the thioredoxin family (12). Thioredoxins have diverse functions, including maintaining the proper reducing environment in cells and participating in apoptotic pathways. These functions are accomplished via thioloxidoreductase reactions mediated by a conserved CXXC catalytic site within a thioredoxin fold (13).

Byrne et al. (14) have shown that the two isoforms of encoding by Nxnl1 have complementary functions. Systemic administration of an adeno-associated virus (AAV) encoding RdCVF improved cone function and delayed cone loss, while RdCVFL increased rhodopsin mRNA and reduced oxidative stress. RdCVFL prevents photo-oxidative damage to the rods (16).

International patent application WO 2016/185037 describes AAV vectors encoding both the short isoform RdCVF and the long isoform RdCVFL and the use of said vectors for treating retinal neurodegenerative pathologies such as retinitis pigmentosa.

A beneficial effect between RdCVF and RdCVFL has been demonstrated (15). On the one hand, RdCVF is produced and secreted by the retinal pigmented epithelium (RPE), protecting the cones by stimulating aerobic glycolysis through the RdCVF receptor at the cell surface of the cones by a non-cell autonomous mechanism (17). On the other hand, RdCVFL, protects the cones against oxidative damage in a cell autonomous manner, due to its thioloxidoreductase function.

Recently, it has been shown that the expression of G protein coupled inwardly rectifying potassium channel 2 (GIRK2) can delay vision loss, by preserving cone light-sensitivity in rd10 and RhoP347S mice and enhance visual acuity (18). Dormant cones, which are cones that have diminished outer segments and thus that became dysfunctional, could be rendered functional again thanks to the expression of GIRK2.

### Summary of the invention

The present invention relates to the combination of a nucleic acid encoding a short isoform of rod-derived cone viability factor (RdCVF), a nucleic acid encoding a long isoform of rod-derived cone viability factor (RdCVFL) and a nucleic acid encoding a G protein-activated Inward Rectifier potassium channel 2 (GIRK2), expressed through one, two or three viral vectors, said vectors may be within a single pharmaceutical composition or within several different pharmaceutical compositions (two or three).

According to a first aspect, the present invention relates to a pharmaceutical composition comprising one or several viral vectors, wherein said one or several viral vectors comprise a nucleic acid encoding RdCVF, a nucleic acid encoding RdCVFL and a nucleic acid encoding GIRK2.

In particular, the pharmaceutical composition comprises a single viral vector.

A second aspect of the invention deals with a viral vector comprising three nucleic acids respectively encoding RdCVF, RdCVFL and GIRK2.

A third aspect of the invention relates to a kit comprising two or three pharmaceutical compositions.

The invention also relates to a pharmaceutical composition, a viral vector or a kit above mentioned, for the treatment of a retinal degenerative disease.

The combination of RdCVF, RdCVFL and GIRK2 described in the present application, allows to both preserve cone cells along with keeping them light responsive. This overcomes the light sensitivity loss that occurs when only trophic factors such as RdCVF and RdCVFL, are used, by implementing an additional approach to increase light sensitivity.

### Detailed description of the invention

The present invention relates to a single or several viral vectors comprising a nucleic acid encoding a short isoform of rod-derived cone viability factor (RdCVF), a nucleic acid encoding a long isoform of rod-derived cone viability factor (RdCVFL) and a nucleic acid encoding a G protein-activated Inward Rectifier potassium channel 2 (GIRK2), and the use thereof. When several, the viral vectors may be in a single or in separate pharmaceutical compositions such as distributed among two or three pharmaceutical compositions.

In one aspect, the present invention relates to a pharmaceutical composition comprising one or several viral vectors, said one or several viral vectors comprising a nucleic acid encoding a short isoform of rod-derived cone viability factor (RdCVF), a nucleic acid encoding a long isoform of rod-derived cone viability factor (RdCVFL) and a nucleic acid encoding a G protein-activated Inward Rectifier potassium channel 2 (GIRK2).

By "one or several viral vectors", it means that said nucleic acid encoding RdCVF, said nucleic acid encoding RdCVFL and said nucleic acid encoding GIRK2 may be comprised in a single vector or in separate vectors such as 2 or 3 vectors.

In a particular embodiment, the pharmaceutical composition comprises three viral vectors respectively comprising a nucleic acid encoding RdCVF, a nucleic acid encoding RdCVFL and a nucleic acid encoding GIRK2.

In a particular embodiment, the pharmaceutical composition comprises two viral vectors wherein the first viral vector comprises a nucleic acid encoding RdCVF and a nucleic acid encoding RdCVFL, and the second viral vector comprises a nucleic acid encoding GIRK2.

In a particular embodiment, the pharmaceutical composition comprises two viral vectors wherein the first viral vector comprises a nucleic acid encoding RdCVF and a nucleic acid encoding GIRK2, and the second viral vector comprises a nucleic acid encoding RdCVFL.

In a particular embodiment, the pharmaceutical composition comprises two viral vectors wherein the first viral vector comprises a nucleic acid encoding GIRK2 and a nucleic acid encoding RdCVFL, and the second viral vector comprises a nucleic acid encoding RdCVF.

In a particular embodiment, the pharmaceutical composition comprises a single viral vector, said single viral vector comprising three nucleic acids respectively encoding RdCVF, RdCVFL and GIRK2.

In another aspect, the present invention relates to a kit comprising two or three pharmaceutical compositions.

When the kit comprises two pharmaceutical compositions, each pharmaceutical composition comprises respectively one viral vector as follows:
- a viral vector comprising a nucleic acid encoding RdCVF and a nucleic acid encoding RdCVFL, and a viral vector comprising a nucleic acid encoding GIRK2;
- a viral vector comprising a nucleic acid encoding RdCVF and a nucleic acid encoding GIRK2, and a viral vector comprising a nucleic acid encoding RdCVFL; or
- a viral vector comprising a nucleic acid encoding GIRK2 and a nucleic acid encoding RdCVFL, and a viral vector comprising a nucleic acid encoding RdCVF.

Thus, in a particular embodiment, the invention relates to a kit comprising two pharmaceutical compositions, wherein:
- the first pharmaceutical composition comprises a viral vector which comprises a nucleic acid encoding RdCVF and a nucleic acid encoding RdCVFL, and
- the second pharmaceutical composition comprises a viral vector which comprises a nucleic acid encoding GIRK2.

When the kit comprises three pharmaceutical compositions, each pharmaceutical composition comprises a single viral vector respectively comprising a nucleic acid encoding RdCVF, a nucleic acid encoding RdCVFL, and a nucleic acid encoding GIRK2.

Thus, in a particular embodiment, the invention relates to kit comprising three pharmaceutical compositions, wherein:
- the first pharmaceutical composition comprises a viral vector, said viral vector comprising a nucleic acid encoding RdCVF,
- the second pharmaceutical composition comprises a viral vector, said viral vector comprising a nucleic acid encoding RdCVFL, and
- the third pharmaceutical composition comprises a viral vector, said viral vector comprising a nucleic acid encoding GIRK2.

In a particular embodiment, the present invention relates to a kit comprising two pharmaceutical compositions, wherein:
- the first pharmaceutical composition comprises a viral vector which comprises a nucleic acid encoding RdCVF and a nucleic acid encoding RdCVFL, and
- the second pharmaceutical composition comprises a viral vector which comprises a nucleic acid encoding GIRK2.

In a particular embodiment, the present invention relates to a kit comprising three pharmaceutical compositions, wherein :
- the first pharmaceutical composition comprises a viral vector, said viral vector comprising a nucleic acid encoding RdCVF,
- the second pharmaceutical composition comprises a viral vector, said viral vector comprising a nucleic acid encoding RdCVFL, and
- the third pharmaceutical composition comprises a viral vector, said viral vector comprising a nucleic acid encoding GIRK2.

The pharmaceutical composition according to the present disclosure may further comprise an excipient pharmaceutically acceptable.

According to the present disclosure, "pharmaceutically acceptable" means that said excipient is generally safe and well tolerated for human or animal use following ocular administration, and should not interfere with the efficacy of the active ingredient (i.e. a viral vector as described in the present disclosure). As example, excipients pharmaceutically acceptable may be isotonic, sterile, saline solutions such as monosodium or disodium phosphate, sodium, potassium, calcium or magnesium chloride and the like or mixtures of such salts. These solutions may further comprise nonionic surfactants such as, e.g., Tween, Pluronic. In a particular embodiment, an excipient pharmaceutically acceptable is a phosphate-buffered saline (PBS) solution or a balanced-salt solution (BSS), even more particularly supplemented with 0.001% Pluronic.

In another aspect, the present invention relates to a viral vector comprising three nucleic acids respectively encoding RdCVF, RdCVFL and GIRK2.

As used herein, the term Rod-derived Cone Viability Factor (RdCVF) refers to the short isoform encoded by the thioredoxin-like 6 (TXNL6) or Nucleoredoxin-like 1 (NXNL1) gene. It encompasses the RdCVF proteins of any animal species. Typically, the RdCVF proteins according to the present invention can be mammalian RdCVF proteins, including, but not limited to human, mice, rats, non-human primates, cats and dogs.

Typically, in mice, the short isoform (RdCVF) is a 109 amino-acid long protein references under Uniprot accession number Q91W38.

In the present application, the short isoform RdCVF is in particular the human short isoform (hRdCVF) as set forth in SEQ ID NO:1.

In particular, said short isoform hRdCVF may be encoded by the nucleic acid as set forth in SEQ ID NO:3.

Alternatively, the nucleic acid encoding the short isoform hRdCVF can be a nucleic acid which differs from SEQ ID NO: 3 but encodes the same amino acid sequence SEQ ID NO:1.

Suitable nucleic acid sequences include but are not limited to:
- polymorphisms of the cDNA encoding human RdCVF;
- combinations of polymorphisms (rare haplotypes) of the cDNA encoding human RdCVF. An example of rare haplotype cDNA is set forth as SEQ ID NO: 6;
- "optimized" sequences in which certain codons are replaced by codons that code for the same amino-acid. Suitable codon-optimized sequences encoding human RdCVF include, but are not limited to, the sequence as set forth in SEQ ID NO: 7;
- homologous sequences. For example, it has been found that the chimpanzee cDNA sequence encoding the short isoform of chimpanzee RdCVF can be used, since it encodes the same amino acid sequence as the human cDNA. The chimpanzee cDNA has the sequence as set forth in SEQ ID NO: 4.

In particular, the short isoform hRdCVF is encoded by a nucleic acid corresponding to a codon-optimized cDNA as set forth in SEQ ID NO:7.

As used herein, the term "RdCVFL" refers to the long isoform encoded by the thioredoxin-like 6 (TXNL6) or Nucleoredoxin-like 1 (NXNL1) gene. It encompasses the RdCVFL proteins of any animal species. Typically, the RdCVFL proteins according to the present invention can be mammalian RdCVFL proteins, including, but not limited to human, mice, rats, non-human primates, cats and dogs.

Typically, in mice, the murine long isoform (RdCVFL) is a 217 amino-acid long protein referenced under Q8VC33.

In the present application, the long isoform RdCVFL is in particular the human long isoform (hRdCVFL) having the sequence referenced under accession number Q96CM4 and as set forth in SEQ ID NO:2.

In particular, said short isoform hRdCVFL may be encoded by the nucleic acid as set forth in SEQ ID NO:5.

Alternatively, the nucleic acid encoding the long isoform hRdCVFL can be a nucleic acid which differs from SEQ ID NO:5 but encodes the same amino acid sequence SEQ ID NO:2.

Suitable nucleic acid sequences include but are not limited to:
- polymorphisms of the cDNA encoding human RdCVFL or combinations thereof;
- "optimized" sequences in which certain codons are replaced by codons that code for the same amino-acid. Suitable codon-optimized sequences encoding human RdCVFL include, but are not limited to, the sequence as set forth in SEQ ID NO:8;
- homologous sequences in other species.

In particular, said short isoform hRdCVFL is encoded by a nucleic acid corresponding to a codon-optimized cDNA as set forth in SEQ ID NO:8.

As used herein, the term "GIRK2" refers to the G protein-activated Inward Rectifier potassium channel 2. It encompasses the GIRK2 proteins of any animal species. Typically, the GIRK2 proteins according to the present invention can be mammalian GIRK2 proteins, including, but not limited to human, mice, rats, non-human primates, cats and dogs.

In particular, the GIRK2 protein may be the human GIRK2 sequence as forth in SEQ ID NO:9.

In particular, said human GIRK2 as forth in SEQ ID NO:9 may be encoded by the nucleic acid as set forth in SEQ ID NO:12.

Alternatively, the nucleic acid encoding the human GIRK2 can be a nucleic acid which differs from SEQ ID NO:12 but encodes the same amino acid sequence SEQ ID NO:9.

In particular, the GIRK2 protein may be the truncated rat GIRK2 sequence as forth in SEQ ID NO:10.

In particular, said truncated rat GIRK2 as forth in SEQ ID NO:10 may be encoded by the nucleic acid as set forth in SEQ ID NO:35.

In particular, the GIRK2 protein may be the mice GIRK2 sequence as forth in SEQ ID NO:11.

Suitable nucleic acid sequences include but are not limited to:
- polymorphisms of the cDNA encoding human GIRK2 or combinations thereof;
- "optimized" sequences in which certain codons are replaced by codons that code for the same amino-acid.
- homologous sequences in other species.

Examples of cDNA sequences of the transgenes RdCVF, RdCVFL and GIRK2 and amino acids sequences are provided at Table 1 and Table 2.

**Table 1: cDNA sequences of transgenes**

| Transgene | cDNA sequence |
|---|---|
| RdCVF | **SEQ ID NO:3** |
| | atggcctccc tgttctctgg ccgcatcctg atccgcaaca atagcgacca ggacgagctg 60 gatacggagg ctgaggtcag tcgcaggctg gagaaccggc tggtgctgct gttctttggt 120 gctggggctt gtccacagtg ccaggccttc gtgcccatcc tcaaggactt cttcgtgcgg 180 ctcacagatg agttctatgt actgcgggcg gctcagctgg ccctggtgta cgtgtcccag 240 gactccacgg aggagcagca ggacctgttc ctcaaggaca tgccaaagaa atggcttttc 300 ctgccctttg aggatgatct gaggaggtga 330 |
| | **SEQ ID NO:6** |
| | atggcctcct tgttctccgg ccgaatcctg atccgaaaca atagtgacca ggatgagctg 60 gatacagaag ctgaggtcag tcgcagactg gagaacaggc tggtccttct gttctttggt 120 gctggggctt gtcctcagtg ccaggccttc gtgcccatcc tcaaggactt ctttgtgcgg 180 ctcacggatg agttctatgt actgcgggca gctcaactgg ctctagtata tgtgtcacag 240 gactccactg aggagcagca ggacctcttc ctgaaggaca tgccaaagaa atggcttttc 300 ctgccctttg aggatgatct gaggaggtag 330 |
| | **SEQ ID NO:7** |
| | atggccagcc tcttctccgg acgcatcctg attcgcaaca attccgacca agacgaactg 60 gataccgagg ccgaagtctc gcggagattg gagaacaggc ttgtgctgct gttctttggc 120 gcgggagcgt gtcctcagtg ccaggctttc gtgccaatcc tgaaggattt cttcgtgcgg 180 ctgactgacg aattctacgt cctccgggcc gcccagctgg cactggtgta cgtgtcccaa 240 gactcaaccg aggaacagca ggatctgttc ctcaaggaca tgcccaaaaa gtggctgttc 300 ctgccgtttg aggacgactt gcggcgctag 330 |
| | **SEQ ID NO:4** |
| | atggcctccc tgttctctgg ccgcatcctg atccgcaaca atagcgacca ggacgaactg 60 gatacggagg ctgaggtcag tcgcaggctg gagaaccggc tggtgctgct gttctttggt 120 gccggggctt gtccacagtg ccaggccttc gtgcccatcc tcaaggactt cttcgtgcgg 180 ctcacagatg agttctatgt actgcgggcg gctcagctgg ccctggtgta cgtgtcccag 240 gactccacgg aggagcagca ggacctgttc ctcaaggaca tgccaaagaa gtggcttttc 300 ctgccctttg aggatgatct gaggaggtga 330 |
| RdCVFL | **SEQ ID NO:5** |
| | atggcctccc tgttctctgg ccgcatcctg atccgcaaca atagcgacca ggacgagctg 60 gatacggagg ctgaggtcag tcgcaggctg gagaaccggc tggtgctgct gttctttggt 120 gctggggctt gtccacagtg ccaggccttc gtgcccatcc tcaaggactt cttcgtgcgg 180 ctcacagatg agttctatgt actgcgggcg gctcagctgg ccctggtgta cgtgtcccag 240 gactccacgg aggagcagca ggacctgttc ctcaaggaca tgccaaagaa atggcttttc 300 ctgccctttg aggatgatct gaggagggac ctcgggcgcc agttctcagt ggagcgcctg 360 ccggcggtcg tggtgctcaa gccggacggg gacgtgctca ctcgcgacgg cgccgacgag 420 atccagcgcc tgggcaccgc ctgcttcgcc aactggcagg aggcggccga ggtgctggac 480 cgcaacttcc agctgccaga ggacctggag gaccaggagc cacggagcct caccgagtgc 540 ctgcgccgcc acaagtaccg cgtggaaaag gcggcgcgag gcgggcgcga ccccggggga 600 gggggtgggg aggagggcgg ggccgggggg ctgttctga 639 |
| | atggcctcac tgttctccgg gcgcatcctc atccgaaaca acagcgatca ggacgaattg 60 gacaccgagg ctgaagtctc ccgccggctg gaaaacaggc tcgtgctcct gttcttcggt 120 gccggagcgt gcccgcagtg ccaagccttc gtcccaattc ttaaggactt ctttgtgcgc 180 ctcactgatg agttttacgt gctccgggca gcgcagctgg ccttggtgta tgtgtcgcaa 240 gattccactg aggaacaaca ggacctgttc ctgaaagaca tgcctaagaa gtggcttttc 300 ctgcccttcg aggacgacct gagaagggac ctgggacgcc agttcagcgt ggaacggctg 360 ccggccgtcg tggtgctgaa gcccgacggg gacgtgctta cccgggatgg cgctgacgaa 420 atccagaggc tgggcaccgc ctgtttcgca aattggcagg aggccgccga agtgctcgac 480 cggaacttcc agctgcccga ggatctggag gaccaggaac ctcggtccct gaccgagtgc 540 ctcagacgcc acaagtaccg cgtggaaaag gccgcgagag gaggacggga cccgggtggc 600 gggggaggcg aagagggcgg agccggtggc ctgttctga 639 **SEQ ID NO:8** |
| GIRK2 | **SEQ ID NO:12** |
| | atggccaagctgaccgagagcatgacaaatgtgctggagggcgacagcatggaccaggatgttgaaagccccgtggccat ccaccagcctaagctgcctaaacaggccagggacgacctgcctagacacatcagcagagacaggaccaagcggaagatc cagagatacgtgcggaaggacggcaagtgcaatgtgcaccacggcaacgtgcgggaaacctacagatacctgaccgacat cttcaccacactggtggacctgaagtggcggttcaacctgctgatcttcgtgatggtgtacacagtgacctggctgttcttcggcat |
| | gatctggtggctgatcgcctacatcagaggcgacatggaccacatcgaggaccctagctggaccccttgcgtgaccaacctga atggcttcgtgtccgccttcctgttcagcatcgagacagagacaaccatcggctacggctacagagtgatcaccgacaagtgcc ctgagggcatcatcctgctgctgatccagtctgtgctgggcagcatcgtgaacgccttcatggtcggatgcatgttcgtgaagatct cccagccaaagaagcgggccgagacactggtgtttagcacccacgccgtgatcagcatgagagatggcaagctgtgcctga tgttcagagtgggcgacctgcggaacagccacatcgtggaagccagcatccgggccaaactgatcaagagcaagcagacc agcgagggcgagttcatccctctgaaccagaccgatatcaacgtgggctactacaccggcgacgacagactgtttctggtgtc ccctctgatcatctcccacgagatcaaccagcagagccccttttgggagatcagcaaggcccagctgccaaaagaggaactg gaaatcgtcgtgatcctggaaggcatggtggaagctaccggcatgacctgtcaggccagaagcagctacatcaccagcgag atcctgtggggctacagattcacccctgtgctgaccctggaagatggcttctacgaggtggactacaactccttccacgagacat acgagacaagcacccctagcctgagcgccaaagagctggctgaactggcctctagagccgagcttcctctgtcttggagcgtg tccagcaagctgaatcagcacgccgagctggaaaccgaggaagaggaaaagaacctggaagaacagaccgagcggaa cggcgacgtggccaacctggaaaatgagagcaaggtgtag |
| | **SEQ ID NO: 35** |
| | ATGGACCAAGACGTGGAAAGCCCAGTGGCCATTCACCAGCCAAAGTTGCCTAAGCAG GCCAGGGATGACCTGCCAAGACACATCAGCCGAGACAGGACCAAAAGGAGAATCCAG AGGTACGTGAGGAAGGATGGGAAGTGTAACGTCCACCACGGCAACGTGCGGGAGAC GTACCGATACCTGACGGACATCTTCACCACCCTGGTGGACCTAAAGTGGAGATTCAAC CTATTGATCTTTGTCATGGTCTACACAGTGACGTGGCTTTTCTTTGGGATGATCTGGTG GCTAATTGCATACATCCGGGGAGATATGGACCACATAGAGGACCCCTCGTGGACTCC CTGTGTTACCAACCTCAACGGGTTTGTCTCCGCTTTTTTATTCTCAATAGAGACAGAAA CCACCATTGGTTATGGCTACAGGGTCATCACGGACAAGTGCCCAGAAGGAATTATTCT CCTCTTAATCCAGTCCGTGTTGGGGTCCATTGTCAACGCCTTCATGGTAGGATGTATG TTTGTGAAAATATCCCAACCCAAGAAGAGGGCAGAGACCCTGGTCTTTTCCACCCATG CGGTAATCTCCATGCGGGATGGGAAACTATGCCTGATGTTCCGGGTAGGGGACTTGA GGAATTCCCACATTGTGGAGGCCTCCATCAGAGCCAAGTTGATCAAGTCCAAACAGAC TTCAGAGGGGGAGTTCATTCCCCTCAACCAGACGGATATCAACGTAGGGTACTACACC GGGGATGACCGACTCTTTCTCGTGTCACCGCTGATTATTAGCCATGAAATTAACCAAC AGAGTCCCTTCTGGGAGATCTCCAAAGCCCAGCTGCCTAAAGAGGAACTGGAGATTG TGGTCATCCTGGAGGGAATGGTGGAAGCCACAGGAATGACGTGCCAAGCTCGAAGCT CCTACGTCACCAGTGAGATCCTGTGGGGTTACCGGTTCACACCAGTCCTGACACTGG AGGACGGGTTCTATGAAGTTGACTACAACAGCTTCCATGAGACCCATGAGACCAGCAC CCCGTCCCTTAGCGCCAAAGAGCTAGCCGAGCTGGCTAACCGGGCAGAGCTGCCCC TGAGCTGGTCTGTGTCCAGCAAACTGAACCAACATGCAGAACTGGAGACGGAAGAGG AAGAGAAGAACCCGGAAGAACTGACAGAGAGGAATGGTGATGTGGCAAACCTAGAGA ATGAGTCCAAAGTGGCG |

**Table 2: Amino acids sequences of transgenes**

| Transgene | Amino acid sequence |
|---|---|
| RdCVF | **SEQ ID NO:1** |
| | MASLFSGRIL IRNNSDQDEL DTEAEVSRRL ENRLVLLFFG AGACPQCQAF VPILKDFFVR LTDEFYVLRA AQLALVYVSQ DSTEEQQDLF LKDMPKKWLF LPFEDDLRR |
| RdCVFL | **SEQ ID NO:2** |
| | MASLFSGRIL IRNNSDQDEL DTEAEVSRRL ENRLVLLFFG AGACPQCQAF VPILKDFFVR LTDEFYVLRA AQLALVYVSQ DSTEEQQDLF LKDMPKKWLF LPFEDDLRRD LGRQFSVERL PAVVVLKPDG DVLTRDGADE IQRLGTACFA NWQEAAEVLD RNFQLPEDLE DQEPRSLTEC LRRHKYRVEK AARGGRDPGG GGGEEGGAGG LF |
| GIRK2 | **SEQ ID NO:9** |
| | |
| | **SEQ ID NO:10** |
| | MDQDVESPVAIHQPKLPKQARDDLPRHISRDRTKRRIQRYVRKDGKCNVHHGNVRETYR YLTDIFTTLVDLKWRFNLLIFVMVYTVTWLFFGMIWWLIAYIRGDMDHIEDPSWTPCVTNLN GFVSAFLFSIETETTIGYGYRVITDKCPEGIILLLIQSVLGSIVNAFMVGCMFVKISQPKKRAE TLVFSTHAVISMRDGKLCLMFRVGDLRNSHIVEASIRAKLIKSKQTSEGEFIPLNQTDINVGY YTGDDRLFLVSPLIISHEINQQSPFWEISKAQLPKEELEIVVILEGMVEATGMTCQARSSYVT SEILWGYRFTPVLTLEDGFYEVDYNSFHETHETSTPSLSAKELAELANRAELPLSWSVSSK LNQHAELETEEEEKNPEELTERNGDVANLENESKVA |
| | **SEQ ID NO:11** |
| | MTMAKLTESM TNVLEGDSMD QDVESPVAIH QPKLPKQARD DLPRHISRDR TKRKIQRYVR KDGKCNVHHG NVRETYRYLT DIFTTLVDLK WRFNLLIFVM VYTVTWLFFG MIWWLIAYIR GDMDHIEDPS WTPCVTNLNG FVSAFLFSIE TETTIGYGYR VITDKCPEGI ILLLIQSVLG SIVNAFMVGC MFVKISQPKK RAETLVFSTH AVISMRDGKL CLMFRVGDLR NSHIVEASIR AKLIKSKQTS EGEFIPLNQT DINVGYYTGD DRLFLVSPLI ISHEINQQSP FWEISKAQLP KEELEIVVIL EGMVEATGMT CQARSSYITS EILWGYRFTP VLTLEDGFYE VDYNSFHETY ETSTPSLSAK ELAELANRAE LPLSWSVSSK LNQHAELETE EEEKNPEELT ERNGDVANLE NESKV |

Typically, the nucleic acids encoding respectively RdCVF, RdCVFL and GIRK2 are under the control of a promoter that allows the expression of said proteins in the target cells.

Suitable promoters can be ubiquitous promoters, such as the Chicken beta actin (CBA) promoter, the chicken beta hybrid (CBh) promoter, the cytomegalovirus (CMV) promoter, the CMV/CBA promoter, CAG promoter.

In particular, the CBh promoter is as set forth in SEQ ID NO: 13.

Suitable promoters can be promoters that enable the expression in the retina, preferably in retinal pigmented epithelial cells and photoreceptor cells such as cones and rods.

In one embodiment, the promoter allows nucleic acids expression in retinal pigmented epithelial cells and/or photoreceptor cells. Non-limiting examples are the rhodopsin kinase (GRK) promoters which target the expression in cones and rods, such as GRK1 promoter, GRK1-93 promoter, IRBP promoter and mCAR promoter.

In particular, the GRK1 promoter is as set forth in SEQ ID NO: 14.

In particular, the GRK1-93 promoter is as set forth in SEQ ID NO: 15.

In one embodiment, the promoter allows nucleic acids expression in cone photoreceptors. Non-limiting examples are the cone-opsin PR1.7 promoter and the ProA7 promoter. In one embodiment, the promoter allows nucleic acids expression in cone photoreceptors. Non-limiting examples are cone-opsin PR1.7 promoter or ProA7 promoter.

In particular, the PR1.7 promoter is as set forth in SEQ ID NO: 16.

In particular, the ProA7 promoter is as set forth in SEQ ID NO: 17.

Table 3 provides particular nucleic acids sequences of the promoters.

**Table 3: Promoters sequences**

| Promoter | DNA sequence |
|---|---|
| CBh | **SEQ ID NO:13** |
| | cgttacataacttacggtaaatggcccgcctggctgaccgcccaacgacccccgcccattgacgtcaatagtaacgccaatagggact ttccattgacgtcaatgggtggagtatttacggtaaactgcccacttggcagtacatcaagtgtatcatatgccaagtacgccccctattga cgtcaatgacggtaaatggcccgcctggcattgtgcccagtacatgaccttatgggactttcctacttggcagtacatctacgtattagtcat cgctattaccatggtcgaggtgagccccacgttctgcttcactctccccatctcccccccctccccacccccaattttgtatttatttattttttaa ttattttgtgcagcgatgggggcggggggggggggggggcgcgcgccaggcggggcggggcggggcgaggggcggggcgggg cgaggcggagaggtgcggcggcagccaatcagagcggcgcgctccgaaagtttccttttatggcgaggcggcggcggcggcggcc ctataaaaagcgaagcgcgcggcgggcgggagtcgctgcgcgctgccttcgccccgtgccccgctccgccgccgcctcgcgccgc ccgccccggctctgactgaccgcgttactcccacaggtgagcgggcgggacggcccttctcctccgggctgtaattagctgagcaaga ggtaagggtttaagggatggttggttggtggggtattaatgtttaattacctggagcacctgcctgaaatcactttttttcaggttgg |
| GRK1 | **SEQ ID NO:14** |
| | gggccccagaagcctggtggttgtttgtccttctcaggggaaaagtgaggcggccccttggaggaaggggccgggcagaatgatcta atcggattccaagcagctcaggggattgtctttttctagcaccttcttgccactcctaagcgtcctccgtgaccccggctgggatttagcctg gtgctgtgtcagccccggtctcccaggggcttcccagtggtccccaggaaccctcgacagggcccggtctctctcgtccagcaagggc agggacgggccacaggccaagggc |
| GRK1-93 | **SEQ ID NO:15** |
| | tctaatcggattccaagcagctcaggggattgtctttttctagcaccttcttgccactcctaagcgtcctccgtgaccccggctgggatttag |
| PR1.7 | **SEQ ID** NO:16 |
| | GGAGGCTGAGGGGTGGGGAAAGGGCATGGGTGTTTCATGAGGACAGAGCTTCCGTTTCATG CAATGAAAAGAGTTTGGAGACGGATGGTGGTGACTGGACTATACACTTACACACGGTAGCG ATGGTACACTTTGTATTATGTATATTTTACCACGATCTTTTTAAAGTGTCAAAGGCAAATGGCC AAATGGTTCCTTGTCCTATAGCTGTAGCAGCCATCGGCTGTTAGTGACAAAGCCCCTGAGTCA AGATGACAGCAGCCCCCATAACTCCTAATCGGCTCTCCCGCGTGGAGTCATTTAGGAGTAGT CGCATTAGAGACAAGTCCAACATCTAATCTTCCACCCTGGCCAGGGCCCCAGCTGGCAGCGA GGGTGGGAGACTCCGGGCAGAGCAGAGGGCGCTGACATTGGGGCCCGGCCTGGCTTGGGT CCCTCTGGCCTTTCCCCAGGGGCCCTCTTTCCTTGGGGCTTTCTTGGGCCGCCACTGCTCCCGC TCCTCTCCCCCCATCCCACCCCCTCACCCCCTCGTTCTTCATATCCTTCTCTAGTGCTCCCTCCAC TTTCATCCACCCTTCTGCAAGAGTGTGGGACCACAAATGAGTTTTCACCTGGCCTGGGGACAC ACGTGCCCCCACAGGTGCTGAGTGACTTTCTAGGACAGTAATCTGCTTTAGGCTAAAATGGG ACTTGATCTTCTGTTAGCCCTAATCATCAATTAGCAGAGCCGGTGAAGGTGCAGAACCTACCG CCTTTCCAGGCCTCCTCCCACCTCTGCCACCTCCACTCTCCTTCCTGGGATGTGGGGGCTGGCA CACGTGTGGCCCAGGGCATTGGTGGGATTGCACTGAGCTGGGTCATTAGCGTAATCCTGGAC AAGGGCAGACAGGGCGAGCGGAGGGCCAGCTCCGGGGCTCAGGCAAGGCTGGGGGCTTCC CCCAGACACCCCACTCCTCCTCTGCTGGACCCCCACTTCATAGGGCACTTCGTGTTCTCAAAG GGCTTCCAAATAGCATGGTGGCCTTGGATGCCCAGGGAAGCCTCAGAGTTGCTTATCTCCCTC TAGACAGAAGGGGAATCTCGGTCAAGAGGGAGAGGTCGCCCTGTTCAAGGCCACCCAGCCA GCTCATGGCGGTAATGGGACAAGGCTGGCCAGCCATCCCACCCTCAGAAGGGACCCGGTGG GGCAGGTGATCTCAGAGGAGGCTCACTTCTGGGTCTCACATTCTTGGATCCGGTTCCAGGCC TCGGCCCTAAATAGTCTCCCTGGGCTTTCAAGAGAACCACATGAGAAAGGAGGATTCGGGCT CTGAGCAGTTTCACCACCCACCCCCCAGTCTGCAAATCCTGACCCGTGGGTCCACCTGCCCCA AAGGCGGACGCAGGACAGTAGAAGGGAACAGAGAACACATAAACACAGAGAGGGCCACAG CGGCTCCCACAGTCACCGCCACCTTCCTGGCGGGGATGGGTGGGGCGTCTGAGTTTGGTTCC CAGCAAATCCCTCTGAGCCGCCCTTGCGGGCTCGCCTCAGGAGCAGGGGAGCAAGAGGTGG GAGGAGGAGGTCTAAGTCCCAGGCCCAATTAAGAGATCAGGTAGTGTAGGGTTTGGGAGCT TTTAAGGTGAAGAGGCCCGGGCTGATCCCACAGGCCAGTATAAAGCGCCGTGACCCTCAGG TGATGCGCCAGGGCCGGCTGCCGTCGGGGACAGGGCTTTCCATAGCCATG |
| ProA7 | **SEQ ID NO:17** |
| | catcctgagagatgagccaggacaaagaaccagtaatagctcctggagcagcacatctgttttgccaggattatcccttggatctcttaa aaccgagaccttgtaatctgaagactcaacttgggctgtacccttaaccttcagctctatgatgcaagtgagtccacaggaccggaggct ttgagatgagcttttcagaagggaggagttggccgcttgctcccagagctccagcacctgcattcttctggctatgtcagaagccagatc atttccctcgttaaaaacaaaaacaaaaaaacaaacaaacaaaatgttagtctttgccctttatctgcctggcaaagcttttaattggcttg atctgtcattccgctagacataaaggggacaatccccggattaggaaggagctctccagctcgggtaaggagtctcaaggcaaggta ggcaagcaccaccggtccgcactctcgcccagcttttacgggaagaagaga |

Typically, the short isoform of the NXNL1 gene is expressed at least by retinal pigmented epithelial cells, and the long isoform of the NXNL1 gene and GIRK2 are expressed at least by cone photoreceptor cells.

Typically, the expression of the nucleic acid encoding RdCVF is driven by a CBh promoter, in particular as set forth in SEQ ID NO:13.

Typically, the expression of the nucleic acid encoding RdCVFL is driven by the ProA7 promoter as set forth in SEQ ID NO:17 or the GRK1 promoter as set forth in SEQ ID NO:14.

Typically, the expression of the nucleic acid encoding GIRK2 is driven by the GRK1-93 promoter as set forth in SEQ ID NO:15.

In a particular embodiment, when a viral vector above described comprises two or three nucleic acids encoding RdCVF, RdCVFL and/or GIRK2, each nucleic acid is under the control of a different promoter.

Typically, when the viral vector comprises three nucleic acids respectively encoding RdCVF, RdCVFL and GIRK2, the nucleic acid encoding RdCVF is under the control of a CBh promoter, the nucleic acid encoding RdCVFL is under the control of a ProA7 promotor or a GRK1 promoter, and the nucleic acid encoding GIRK2 is under the control of a GRK1-93 promoter.

In a particular embodiment, when a viral vector above described comprises two or three nucleic acids encoding RdCVF, RdCVFL and/or GIRK2, at least two nucleic acids may be under the control of a same promoter and linked by a nucleic acid sequence encoding a 2A self-cleaving peptide.

2A self-cleaving peptides, or 2A peptides, is a class of 18-22 amino acids-long peptides, which can induce ribosomal skipping during translation of a protein in a cell. These peptides share a core sequence motif of DxExNPGP (SEQ ID NO:18).

As examples of 2A self-cleaving peptide P2A (SEQ ID NO:19), T2A (SEQ ID NO:20), E2A (SEQ ID NO:21) and F2A (SEQ ID NO:22) may be cited. Sequences are provided at Table 4.

**Table 4: 2A self-cleaving peptides**

| | |
|---|---|
| P2A | SEQ ID NO:19: GSGATNFSLLKQAGOVEENPGP |
| | SEQ ID NO:23 ggaagcggagccacgaacttctctctgttaaagcaagcaggagatgttgaagaaaaccccgggcct |
| T2A | SEQ ID NO:20 GSGEGRGSLL TCGDVEENPGP |
| E2A | SEQ ID NO:21 GSGQCTNYALLKLAGDVESNPGP |
| F2A | SEQ ID NO:22 GSGVKQTLNFDLLKLAGDVESNPGP |

In particular, the 2A self-cleaving peptide is the P2A peptide as set forth in SEQ ID NO: 19. In a particular embodiment, this P2A peptide is encoded by the nucleic acid sequence as set forth in SEQ ID NO:23.

In a particular embodiment, the viral vector above described comprises three nucleic acids respectively encoding RdCVF, RdCVFL and GIRK2, wherein the nucleic acid encoding RdCVF is under control of a promoter, in particular a CBh promoter, the nucleic acid encoding RdCVFL is under control of a promoter, in particular a GRK1 promoter, and it is linked to the nucleic acid encoding GIRK2 by a nucleic acid sequence encoding a 2A self-cleaving peptide, in particular the P2A peptide.

In a particular embodiment, the viral vector above described comprises three nucleic acids respectively encoding RdCVF, RdCVFL and GIRK2, wherein the nucleic acid encoding RdCVF is under control of a promoter, in particular a CBh promoter, and it is linked to the nucleic acid encoding RdCVFL by a nucleic acid sequence encoding a 2A self-cleaving peptide, this latter being linked to the nucleic acid encoding GIRK2 by a nucleic acid sequence encoding a 2A self-cleaving peptide, in particular the P2A peptide.

In a particular embodiment, the viral vector above described comprises three nucleic acids respectively encoding RdCVF, RdCVFL and GIRK2, wherein the nucleic acid encoding RdCVF is under control of a promoter, in particular a CBh promoter, and it is linked to the nucleic acid encoding GIRK2 by a nucleic acid sequence encoding a 2A self-cleaving peptide, in particular the P2A peptide, this latter being linked to the nucleic acid encoding RdCVFL by a nucleic acid sequence encoding a 2A self-cleaving peptide, in particular the P2A peptide.

As in all AAV vectors, the viral vector according to the invention comprises an ITR sequence in 5' and an ITR sequence in 3'.

In a particular embodiment wherein the viral vector comprises two or three nucleic acids encoding RdCVF, RdCVFL and/or GIRK2 and wherein at least two nucleic acids are linked by a nucleic acid sequence encoding a 2A self-cleaving peptide, the nucleic acids are in any order following the 5'ITR. More particularly, the first nucleic acid following the 5'ITR is the nucleic acid encoding RdCVF.

In a particular embodiment wherein the viral vector comprises three nucleic acids encoding RdCVF, RdCVFL and/or GIRK2 and wherein the nucleic acids are all linked by a nucleic acid sequence encoding a 2A self-cleaving peptide, the first nucleic acid following the 5'ITR is the nucleic acid encoding RdCVF and the second is the nucleic acid encoding RdCVFL.

In a particular embodiment wherein the viral vector comprises three nucleic acids encoding RdCVF, RdCVFL and/or GIRK2 and wherein the nucleic acids are all linked by a nucleic acid sequence encoding a 2A self-cleaving peptide, the first nucleic acid following the 5'ITR is the nucleic acid encoding RdCVF and the second is the nucleic acid encoding GIRK2.

In a particular embodiment, the viral vector described in the present disclosure further comprises a posttranscriptional regulatory element (PRE). This is a DNA sequence that, when transcribed, creates a tertiary structure enhancing expression. This type of sequence is commonly used in molecular biology to increase expression of genes delivered by viral vectors.

In a more particular embodiment, it is a Woodchuck Hepatitis Virus PRE (WPRE), more particularly as set forth in SEQ ID NO:24.
SEQ ID NO:24

cgataatcaacctctggattacaaaatttgtgaaagattgactggtattcttaactatgttgctccttttacgctatgtggatacgctgctttaatgc
ctttgtatcatgctattgcttcccgtatggctttcattttctcctccttgtataaatcctggttgctgtctctttatgaggagttgtggcccgttgtcaggc
aacgtggcgtggtgtgcactgtgtttgctgacgcaacccccactggttggggcattgccaccacctgtcagctcctttccgggactttcgctttc
cccctccctattgccacggcggaactcatcgccgcctgccttgcccgctgctggacaggggctcggctgttgggcactgacaattccgtggt
gttgtcggggaagctgacgtcctttccatggctgctcgcctgtgttgccacctggattctgcgcgggacgtccttctgctacgtcccttcggccct
caatccagcggaccttccttcccgcggcctgctgccggctctgcggcctcttccgcgtcttcgccttcgccctcagacgagtcggatctcccttt
gggccgcctccccgcatcgg

As used herein, the term "viral vector" has its general meaning in the art. In particular, it encompasses a vector derived from an adeno-associated virus (AAV), a herpesvirus (e.g., herpes simplex virus (HSV)), an adenovirus, a retrovirus, a lentivirus, or a vaccinia/poxvirus.

In the present disclosure, the expression "adeno-associated virus vector" or "AAV vector" has its general meaning in the art.

AAV and AAV vectors have been extensively described in the art as suitable vectors for gene delivery.

Indeed, AAV are non-pathogenic and display a broad range of tissue specificity, depending of their serotype. Typically, AAV according to the present invention are AAV able to target retinal cells.

Examples of AAV serotypes include, but are not limited to, AAV8, AAV2, AAV5, AAV7, AAV9.

In a particular embodiment, the AAV is an AAV8 (also referred to as AAV2/8).

In one embodiment, the AAV and the AAV vector according to the present invention is obtained according to the method described in international patent application WO2012/158757.

In a particular embodiment, the AAV capsid is obtained according to the method described in patent application US9193956B2.

The above cited AAVs may comprise a variant VP1 capsid protein, wherein the variant AAV capsid protein comprises an insertion peptide of from 7 amino acids to 11 amino acids in the GH loop of said capsid protein relative to a corresponding parental AAV capsid protein.

Said insertion peptide may be as set forth in SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33 as described in WO2019/077159 and at Table 5 below.

The insertion peptide may also be as set forth in SEQ ID NO:34 (28).

**Table 5: Insertion peptide sequences**

| | |
|---|---|
| SEQ ID NO:25 | Leu Gly Glu Thr Thr Arg Pro |
| SEQ ID NO:26 | Asn Glu Thr Ile Thr Arg Pro |
| SEQ ID NO:27 | Lys Ala Gly Gln Ala Asn Asn |
| SEQ ID NO:28 | Lys Asp Pro Lys Thr Thr Asn |
| SEQ ID NO:29 | Lys Asp Thr Asp Thr Thr Arg |
| SEQ ID NO:30 | Arg Ala Gly Gly Ser Val Gly |
| SEQ ID NO:31 | Ala Val Asp Thr Thr Lys Phe |
| SEQ ID NO:32 | Ser Thr Gly Lys Val Pro Asn |
| SEQ ID NO:33 | Ala Ala Leu Glv Glu Thr Thr Arg Pro Ala |
| SEQ ID NO:34 | Leu Ala Leu Gly Glu Thr Thr Arg Pro |

Further examples of AAV are thus AAV serotypes as above mentioned, comprising such an insertion peptide.

The insertion peptide may also be as set forth in SEQ ID NO:37 (LAISDQTKHA). Thus, AAV serotypes as above mentioned may comprise an insertion peptide as set forth in SEQ ID NO:37.

In a particular embodiment, the above cited AAVs may comprise a variant AAV capsid protein as set forth in SEQ ID NO:36.

In a particular embodiment, the above cited AAVs may comprise a variant AAV capsid protein as described in SEQ ID NO:42 of WO2019104279A1.

In another aspect, the present invention deals with a pharmaceutical composition above described, a kit above described, or a viral vector above described for the treatment of a retinal degenerative disease.

In the context of the invention, the term "treating" or "treatment", as used herein, means reversing, alleviating, inhibiting the progress of, or preventing the disorder or condition to which such term applies, or one or more symptoms of such disorder or condition (e.g., retinal degenerative diseases).

In the present application, the term "retinal degenerative disease" encompasses all diseases associated with rods and cones degeneration. It encompasses different subgroups of pathologies: Rod-cone dystrophies, Cone dystrophies, Cone-rod dystrophies, and atrophic age-related macular degeneration.

Thus, in a particular embodiment, the present invention relates to a pharmaceutical composition above described, a kit above described, or a viral vector above described for treatment of a retinal degenerative disease, wherein said retinal degenerative disease is a rod-cone dystrophy, a cone dystrophy, a cone-rod dystrophy or an atrophic age-related macular degeneration.

Retinal degenerative diseases include but are not limited to retinitis pigmentosa, age-related macular degeneration, Bardet-Biedel syndrome, Bassen-Kornzweig syndrome, Best disease, choroideremia, gyrate atrophy, Leber congenital amaurosis, Refsum disease, Stargardt disease or Usher syndrome.

In a particular embodiment, the retinal degenerative disease is a rod-cone dystrophy, more particularly the retinitis pigmentosa.

In a particular embodiment, the pharmaceutical composition, the kit or the viral vector is administered to a patient in need by subretinal injection, intravitreal and suprachoroidal injection. The delivery of the vector may be submacular or subfoveal or a distal bleb from the fovea without detaching said region.

When the kit is administered, the pharmaceutical compositions of the kit may be administered simultaneously or separately over time.

The stoichiometry between the pharmaceutical compositions of the kit can be varied to obtain optimal protection and functional restoration.

The present invention also relates to a method for the treatment of a retinal degenerative disease comprising a step of administering to a patient in need of a therapeutically effective amount of a pharmaceutical composition above described, of a viral vector above described, or of pharmaceutical compositions comprised in kits above described.

The term "therapeutically effective amount" as used herein means an amount sufficient to achieve a desired biological effect, in this case increasing the neuron viability, and thus to reduce symptoms or progression of the disease in a patient in need. It is understood that the effective dosage will be dependent upon the age, sex, health, and weight of the recipient, kind of concurrent treatment, if any, frequency of treatment, and the nature of the effect desired. However, the preferred dosage can be tailored to the individual subject, as is understood and determinable by one of skill in the art, without undue experimentation.

The present invention also relates to the use of a pharmaceutical composition above described, a kit above described, or a viral vector above described, in the treatment of a retinal degenerative disease.

### Brief Description of Drawings

**Fig. 1**

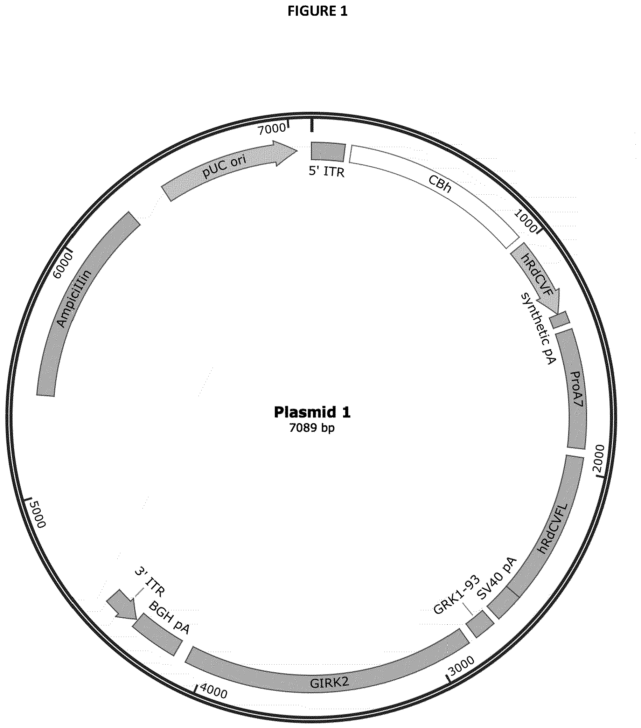

   [Fig. 1] is a map of plasmid 1: CBh-hRdCVF-ProA7-hRdCVFL-GRK1-93-GIRK2
**Fig. 2**

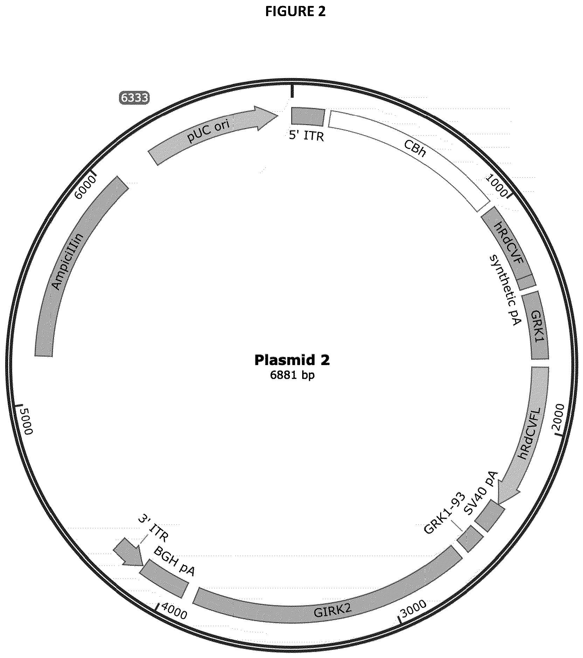

   [Fig. 2] is a map of plasmid 2: CBh-hRdCVF-GRK1-hRdCVFL-GRK1-93-GIRK2
**Fig. 3**

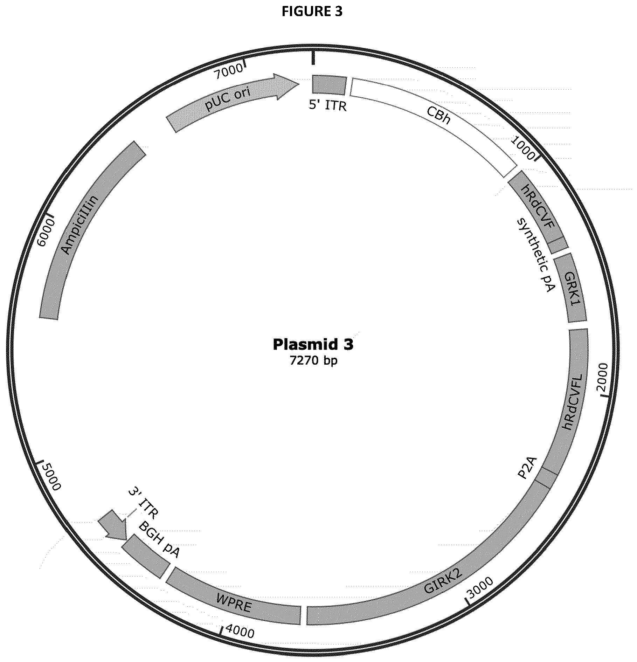

   [Fig. 3] is a map of plasmid 3: CBh-hRdCVF-GRK1-hRdCVFL-P2A-GIRK2 (+WPRE)
**Fig. 4**

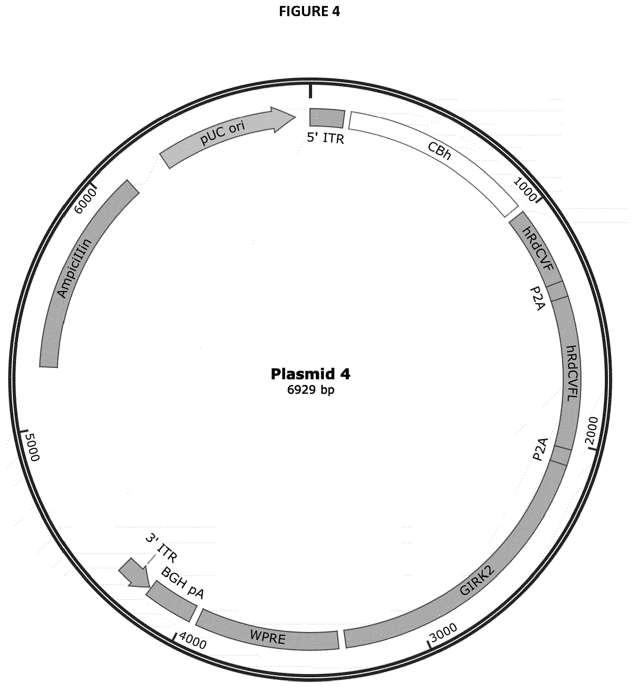

   [Fig. 4] is a map of plasmid 4: CBh-hRdCVF-P2A-hRdCVFL-P2A-GIRK2- (+WPRE)
**Fig. 5**

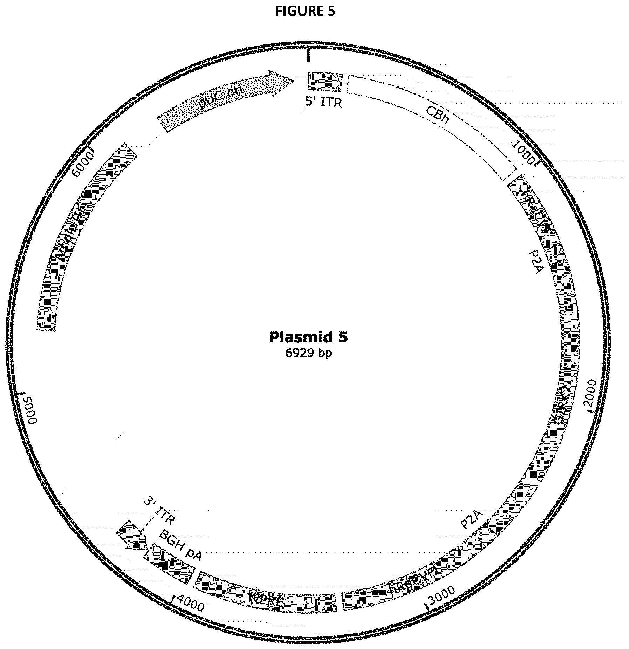

   [Fig. 5] is a map of plasmid 5: CBh-hRdCVF-P2A-GIRK2-P2A-hRdCVFL- (+WPRE)
**Fig. 6**

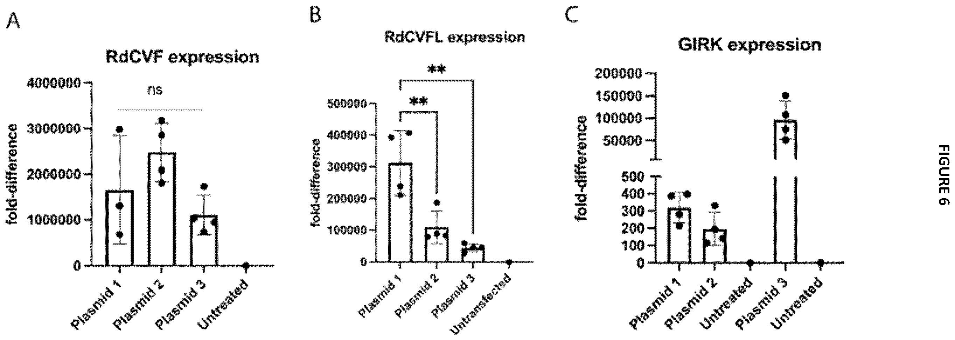

   [Fig. 6] shows the RdCVF, RdCVFL and GIRK expression 48h after transfection of 661W cells with Plasmids 1, 2 or 3. **(A)** RdCVF transcript was quantified using qPCR with the same set of primers for the three plasmids. **(B)** RdCVFL transcript was quantified using qPCR with the same set of primers for the three plasmids. **(C)** GIRK2 transcript was quantified using qPCR with the same set of primers for plasmids 1 and 2. GIRK2 transcript was quantified with a second set of primers that are specific for Plasmid 3.

### Examples

### Constructs design

Different plasmids are designed.

### Plasmid 1: CBh-hRdCVF-ProA7-hRdCVFL-GRK1-93-GIRK2

Plasmid 1 comprises a nucleic acid of SEQ ID NO:7 encoding the human short isoform RdCVF as set forth in SEQ ID NO:1, under control of a promoter CBh as set forth in SEQ ID NO:13; a nucleic acid of SEQ ID NO:8 encoding the human long isoform RdCVFL as set forth in SEQ ID NO:2, under control of a promoter ProA7 as set forth in SEQ ID NO: 17; and a nucleic acid of SEQ ID NO: 35 encoding the truncated rat GIRK2 as set forth in SEQ ID NO:10, under control of a promoter GRK1-93 as set forth in SEQ ID NO:15.

### Plasmid 2: CBh-hRdCVF-GRK1-hRdCVFL-GRK1-93-GIRK2

Plasmid 2 comprises a nucleic acid of SEQ ID NO:7 encoding the human short isoform RdCVF as set forth in SEQ ID NO:1, under control of a promoter CBh as set forth in SEQ ID NO:13; a nucleic acid of SEQ ID NO:8 encoding the human long isoform RdCVFL as set forth in SEQ ID NO:2, under control of a promoter GRK1 as set forth in SEQ ID NO:14; and a nucleic acid of SEQ ID NO: 35 encoding the truncated rat GIRK2 as set forth in SEQ ID NO:10, under control of a promoter GRK1-93 as set forth in SEQ ID NO:15.

### Plasmid 3: CBh-hRdCVF-GRK1-hRdCVFL-P2A-GIRK2 - (+WPRE)

Plasmid 3 comprises a nucleic acid of SEQ ID NO:7 encoding the human short isoform RdCVF as set forth in SEQ ID NO:1, under control of a promoter CBh as set forth in SEQ ID NO:13; a nucleic acid of SEQ ID NO:8 encoding the human long isoform RdCVFL as set forth in SEQ ID NO:2, under control of a promoter GRK1 as set forth in SEQ ID NO:14; which is linked to a nucleic acid of SEQ ID NO: 35 encoding the truncated rat GIRK2 as set forth in SEQ ID NO:10 by a nucleic acid sequence encoding a 2A self-cleaving peptide (P2A) as set forth in SEQ ID NO:23. Plasmid 3 further comprises a WPRE sequence as set forth in SEQ ID NO:24.

### Plasmid 4: CBh-hRdCVF-P2A-hRdCVFL-P2A-GIRK2- (+WPRE)

Plasmid 4 comprises a nucleic acid of SEQ ID NO:7 encoding the human short isoform RdCVF as set forth in SEQ ID NO:1, under control of a promoter CBh as set forth in SEQ ID NO:13; this latter nucleic acid being linked by a nucleic acid sequence encoding a 2A self-cleaving peptide (P2A) as set forth in SEQ ID NO:23, to a nucleic acid of SEQ ID NO:8 encoding the human long isoform RdCVFL as set forth in SEQ ID NO:2, this latter being itself linked to a nucleic acid of SEQ ID NO:35 encoding the truncated rat GIRK2 as set forth in SEQ ID NO:10, by a nucleic acid sequence encoding a 2A self-cleaving peptide (P2A) as set forth in SEQ ID NO:23. Plasmid 4 further comprises a WPRE sequence as set forth in SEQ ID NO:24.

### Plasmid 5: CBh-hRdCVF-P2A-GIRK2-P2A-hRdCVFL- (+WPRE)

Plasmid 5 comprises a nucleic acid of SEQ ID NO:7 encoding the human short isoform RdCVF as set forth in SEQ ID NO:1, under control of a promoter CBh as set forth in SEQ ID NO:13; this latter nucleic acid being linked by a nucleic acid sequence encoding a 2A self-cleaving peptide (P2A) as set forth in SEQ ID NO:23, to a nucleic acid of SEQ ID NO:35 encoding the truncated rat GIRK2 as set forth in SEQ ID NO:10, this latter being itself linked to a nucleic acid of SEQ ID NO:8 encoding the human long isoform RdCVFL as set forth in SEQ ID NO:2, by a nucleic acid sequence encoding a 2A self-cleaving peptide (P2A) as set forth in SEQ ID NO:23. Plasmid 5 further comprises a WPRE sequence as set forth in SEQ ID NO:24.

### In vitro tests

661W cells were transfected using Lipofectamine reagent (Thermo Fisher) with the plasmids 1, 2 and 3 above described. Each construct was tested in triplicates (three wells/plasmid).

The RNA expression of each of RdCVF, RdCVFL and GIRK2 transgenes was then quantified by RT-qPCR. For that, 48 hours after transfection, RNAs were extracted using the Nucleospin RNA kit (Macherey-Nagel). RNAs were then treated with Turbo-DNAse kit (Thermo Fisher) to remove DNA. Reverse transcriptions were then performed the Supercript Reverse Transcriptase kit (Thermo Fisher) using oligodT primers. Finally, cDNA was amplified with qPCR (SYBR Green, Thermo Fisher) using plasmid-specific primers, that do not amplify endogenous 661W cells transcripts.

RdCVF transcript was quantified using qPCR with the same set of primers for the three plasmids. RdCVFL transcript was quantified using qPCR with the same set of primers for the three plasmids. GIRK2 transcript was quantified using qPCR with the same set of primers for plasmids 1 and 2. GIRK2 transcript was quantified with a second set of primers that are specific for Plasmid 3.

Results of transgenes expression is shown on Figure 6.

The 3 tested plasmids are able to express the three transgenes.

### In vivo tests

Plasmids 1 to 5 are packaged into AAV8, and then are tested in vivo in rd10 mice model by subretinal injection.

Each vector is tested in the left eye of 15 homozygous rd10 mice aged P15. Two doses are tested:
- Group 1: 5e + 8 vg/eye in 1 µl (vg = viral genome);
- Group 2: 5e + 7 vg/eye in 1 µl.

The right eye is treated with 1 µl of a phosphate-buffered saline solution (PBS) with pluronic (poloxamers) at 0.001%, as control.

Output measurements are performed at 15 days, 30 days, 2 months and 3 months post-injection.

Electroretinogram (ERG), optical coherence tomography (OCT) analysis and optokinetic reflex are carried out, along with histological analysis of retinas. Examination of the eye fundus are also performed in mice to check the expression of the transgene.

### Cited references

1. Buch H et al. Prevalence and causes of visual impairment and blindness among 9980 Scandinavian adults: the Copenhagen City Eye Study. Ophthalmology. 2004;111(1):53-61.
2. Bramall AN, Wright AF, Jacobson SG, Mclnnes RR. The genomic, biochemical, and cellular responses of the retina in inherited photoreceptor degenerations and prospects for the treatment of these disorders. Annu Rev Neurosci. 2010;33(1):441-472.
3. Punzo C, Kornacker K, Cepko CL. Stimulation of the insulin/mTOR pathway delays cone death in a mouse model of retinitis pigmentosa. Nat Neurosci. 2009;12(1):44-52.
4. Léveillard T et al. Identification and characterization of rod-derived cone viability factor. Nat Genet. 2004;36(7):755-759.
5. Mohand-Said S et al. Normal retina releases a diffusible factor stimulating cone survival in the retinal degeneration mouse. Proc Natl Acad Sci U S A. 1998;95(14):8357-8362.
6. Mohand-Said S et al. Photoreceptor transplants increase host cone survival in the retinal degeneration (rd) mouse. Ophthalmic Res. 1997;29(5):290-297.
7. Mohand-Said S, Hicks D, Dreyfus H, Sahel J-A. Selective transplantation of rods delays cone loss in a retinitis pigmentosa model. Arch Ophthalmol. 2000;118(6):807-811.
8. Wang XW, Tan BZ, Sun M, Ho B, Ding JL. Thioredoxin-like 6 protects retinal cell line from photooxidative damage by upregulating NF-kappaB activity. Free Radic Biol Med. 2008;45(3):336-344.
9. Yang Y et al. Functional Cone Rescue by RdCVF Protein in a Dominant Model of Retinitis Pigmentosa. Mol Ther. 2009;17(5):787-795.
10. Cronin T et al. The disruption of the rod-derived cone viability gene leads to photoreceptor dysfunction and susceptibility to oxidative stress. Cell Death Differ. 2010;17(7):1199-1210.
11. Brennan LA, Lee W, Kantorow M. TXNL6 is a novel oxidative stress-induced reducing system for methionine sulfoxide reductase a repair of α-crystallin and cytochrome C in the eye lens. PLoS ONE. [published online ahead of print: 2010].
12. Funato Y, Miki H. Nucleoredoxin, a Novel Thioredoxin Family Member Involved in Cell Growth and Differentiation. Antioxid Redox Signal. 2007;9(8):1035-1058.
13. Lillig CH, Holmgren A. Thioredoxin and Related Molecules-From Biology to Health and Disease. Antioxid Redox Signal. 2007;9(1):25-47.
14. Byrne LC, Dalkara D, Luna G, Fisher SK, Clérin E, Sahel JA, Léveillard T, Flannery JG. Viral-mediated RdCVF and RdCVFL expression protects cone and rod photoreceptors in retinal degeneration. J Clin Invest. 2015 125(1):105-16.
15. Mei X., Chaffiol, A., Kole, C., Yang Y., Millet-Puel G., Clérin E., Aït-Ali N., Bennett, J., Dalkara D., Sahel JA, Duebel, J., Léveillard T., The thioredoxin encoded by the Rod-derived Cone Viability Factor gene protects cone photoreceptors against oxidative stress. Antioxid Redox Signal. 2016 May 12.
16. Elachouri G, Lee-Rivera I, Clerin E, Argentini M, Fridlich R, Blond F, Ferracane V, Yang Y, Raffelsberger W, Wan J, Bennett J, Sahel JA, Zack DJ, Leveillard T: Thioredoxin rod-derived cone viability factor protects against photooxidative retinal damage. Free radical biology & medicine 2015, 81:22-29.
17. Ait-Ali N, Fridlich R, Millet-Puel G, Clerin E, Delalande F, Jaillard C, Blond F, Perrocheau L, Reichman S, Byrne LC, Olivier-Bandini A, Bellalou J, Moyse E, Bouillaud F, Nicol X, Dalkara D, van Dorsselaer A, Sahel JA, Leveillard T: Rod-derived cone viability factor promotes cone survival by stimulating aerobic glycolysis. Cell 2015, 161(4):817-832.
18. Simon C.-J. et al. Poster entitled G-protein gated K+ channel-mediated vision restoration in Rod-Cone dystrophy. ESGCT Barcelona, 24 October 2019.
19. Dryja et al., Gene-based approach to human gene-phenotype correlations. Proc Natl Acad Sci U S A. 1997 Oct 28;94(22):12117-21.
20. Sinha R, Hoon M, Baudin J, Okawa H, Wong ROL, Rieke F. 2017. Cellular and Circuit Mechanisms Shaping the Perceptual Properties of the Primate Fovea. Cell. 168(3):413-426.
21. Leveillard, T and Sahel, JA (2010). "Rod-derived cone viability factor for treating blinding diseases: from clinic to redox signaling." Sci Transl Med 2(26): 26ps16.
22. Lindblad, AS; Lloyd, PC; Clemons, TE; Gensler, GR; Ferris FL, 3rd; Klein, ML; Armstrong, JR; Age-Related Eye Disease Study Research, Group. (September 2009). "Change in area of geographic atrophy in the Age-Related Eye Disease Study: AREDS report number 26" (PDF). Archives of Ophthalmology. 127 (9): 1168-74. doi:10.1001/archophthalmol.2009.198.
23. Sunness, JS (3 November 1999). "The natural history of geographic atrophy, the advanced atrophic form of age-related macular degeneration". Molecular Vision. 5: 25.
24. Bonilha, Vera L (2008). "Age and disease-related structural changes in the retinal pigment epithelium". Clinical Ophthalmology (Auckland, N.Z.). 2 (2): 413-424.
25. Lindner, Moritz; Fleckenstein, Monika; Schmitz-Valckenberg, Steffen; Holz, Frank G. (2018), "Atrophy, Geographic", Encyclopedia of Ophthalmology, Springer Berlin Heidelberg, pp. 207-209.
26. Wong, Wan Ling; Su, Xinyi; Li, Xiang; Cheung, Chui Ming G; Klein, Ronald; Cheng, Ching-Yu; Wong, Tien Yin (February 2014). "Global prevalence of age-related macular degeneration and disease burden projection for 2020 and 2040: a systematic review and meta-analysis". The Lancet Global Health. 2 (2): e106-e116.
27. Rudnicka, Alicja R.; Kapetanakis, Venediktos V.; Jarrar, Zakariya; Wathern, Andrea K.; Wormald, Richard; Fletcher, Astrid E.; Cook, Derek G.; Owen, Christopher G. (July 2015). "Incidence of Late-Stage Age-Related Macular Degeneration in American Whites: Systematic Review and Meta-analysis". American Journal of Ophthalmology. 160 (1): 85-93.e3.
28. Dalkara et al., 2013. "In vivo-directed evolution of a new adeno-associated virus for therapeutic outer retinal gene delivery form the vitreous". Science Translational Medecine, Vol 5, issue 189.

## Claims

1. A pharmaceutical composition comprising one or several viral vectors, wherein said one or several viral vectors comprise a nucleic acid encoding a short isoform of rod-derived cone viability factor (RdCVF), a nucleic acid encoding a long isoform of rod-derived cone viability factor (RdCVFL) and a nucleic acid encoding a G protein-activated Inward Rectifier potassium channel 2 (GIRK2).

2. A pharmaceutical composition according to claim 1, wherein said composition comprises a first viral vector comprising a nucleic acid encoding RdCVF and a nucleic acid encoding RdCVFL, and a second viral vector comprising a nucleic acid encoding GIRK2.

3. A pharmaceutical composition according to claim 1, wherein said composition comprises three viral vectors respectively comprising a nucleic acid encoding RdCVF, a nucleic acid encoding RdCVFL, and a nucleic acid encoding GIRK2.

4. A pharmaceutical composition according to claim 1, wherein said pharmaceutical composition comprises a single viral vector, said single viral vector comprising three nucleic acids respectively encoding RdCVF, RdCVFL and GIRK2.

5. A viral vector comprising three nucleic acids respectively encoding RdCVF, RdCVFL and GIRK2.

6. A kit comprising two pharmaceutical compositions, wherein:
- the first pharmaceutical composition comprises a viral vector which comprises a nucleic acid encoding RdCVF and a nucleic acid encoding RdCVFL, and
- the second pharmaceutical composition comprises a viral vector which comprises a nucleic acid encoding GIRK2.

7. A kit comprising three pharmaceutical compositions, wherein :
- the first pharmaceutical composition comprises a viral vector, said viral vector comprising a nucleic acid encoding RdCVF,
- the second pharmaceutical composition comprises a viral vector, said viral vector comprising a nucleic acid encoding RdCVFL, and
- the third pharmaceutical composition comprises a viral vector, said viral vector comprising a nucleic acid encoding GIRK2.

8. A pharmaceutical composition according to any one of claims 1 to 4, a viral vector according to claim 5, or a kit according to claim 6 or 7, wherein said RdCVF is the human short isoform hRdCVF as set forth in SEQ ID NO:1.

9. A pharmaceutical composition according to any one of claims 1 to 4 or 8, or a viral vector according to claim 5 or 8, or a kit according to any one of claims 6 to 8, wherein said RdCVFL is the human long isoform hRdCVFL as set forth in SEQ ID NO:2.

10. A pharmaceutical composition according to any one of claims 1 to 4 or 8 to 9, or a viral vector according to any one of claims 5, 8 or 9, or a kit according to any one of claims 6 to 9, wherein said GIRK2 is the human GIRK2 sequence as forth in SEQ ID NO:9.

11. A pharmaceutical composition according to any one of claims 1 to 4 or 8 to 10, or a viral vector according to any one of claims 5 or 8 to 10, or a kit according to any one of claims 6 to 10, for use in the treatment of a retinal degenerative disease.

12. A pharmaceutical composition according to any one of claims 1 to 4 or 8 to 10, or a viral vector according to any one of claims 5 or 8 to 10, or a kit according to any one of claims 6 to 10, for use according to claim 11, wherein said retinal degenerative disease is a rod-cone dystrophy, a cone dystrophy, a cone-rod dystrophy or an atrophic age-related macular degeneration.

13. A pharmaceutical composition according to claims 1 to 4 or 8 to 10, or a viral vector according to any one of claims 5, 8 to 10, or a kit according to any one of claims 6 to 10, for use according to claim 11, wherein said retinal degenerative disease is selected in the group consisting of retinitis pigmentosa, age-related macular degeneration, Bardet-Biedel syndrome, Bassen-Kornzweig syndrome, Best disease, choroideremia, gyrate atrophy, Leber congenital amaurosis, Refsum disease, Stargardt disease or Usher syndrome.

14. A pharmaceutical composition according to any one of claims 1 to 4 or 8 to 10, a viral vector according to any one of claims 5, or 8 to 10, or a kit according to any one of claims 6 to 10, for use according to claim 11, wherein said retinal degenerative disease is the retinitis pigmentosa.

15. A pharmaceutical composition according to any one of claims 1 to 4 or 8 to 10, a viral vector according to any one of claims 5 or 8 to 10, or a kit according to any one of claims 6 to 10, for use according to any of claims 11 to 14, wherein said pharmaceutical composition or said viral vector is administered by subretinal injection, intravitreal injection or suprachoroidal injection.

## Patentansprüche

1. Pharmazeutische Zusammensetzung, umfassend einen oder mehrere virale Vektoren, wobei der eine oder die mehreren viralen Vektoren eine Nukleinsäure, welche eine kurze Isoform des Rod-derived Cone Viability Factor (RdCVF) codiert, eine Nukleinsäure, welche eine lange Isoform des Rod-derived Cone Viability Factor (RdCVFL) codiert, und eine Nukleinsäure, welche einen G-Protein-aktivierten Inward-Rectifier-Kaliumkanal 2 (GIRK2) codiert, umfasst.

2. Pharmazeutische Zusammensetzung nach Anspruch 1, wobei die Zusammensetzung einen ersten viralen Vektor, umfassend eine Nukleinsäure, welche RdCVF codiert, und eine Nukleinsäure, welche RdCVFL codiert, und einen zweiten viralen Vektor, umfassend eine Nukleinsäure, welche GIRK2 codiert, umfasst.

3. Pharmazeutische Zusammensetzung nach Anspruch 1, wobei die Zusammensetzung drei virale Vektoren umfasst, welche jeweils eine Nukleinsäure, welche RdCVF codiert, eine Nukleinsäure, welche RdCVFL codiert, und eine Nukleinsäure, welche GIRK2 codiert, umfassen.

4. Pharmazeutische Zusammensetzung nach Anspruch 1, wobei die pharmazeutische Zusammensetzung einen einzelnen viralen Vektor umfasst, wobei der einzelne virale Vektor drei Nukleinsäuren umfasst, welche jeweils RdCVF, RdCVFL und GIRK2 codieren.

5. Viraler Vektor, umfassend drei Nukleinsäuren, welche jeweils RdCVF, RdCVFL und GIRK2 codieren.

6. Kit, umfassend zwei pharmazeutische Zusammensetzungen, wobei:
- die erste pharmazeutische Zusammensetzung einen viralen Vektor umfasst, welcher eine Nukleinsäure, welche RdCVF codiert, und eine Nukleinsäure, welche RdCVFL codiert, umfasst, und
- die zweite pharmazeutische Zusammensetzung einen viralen Vektor umfasst, welcher eine Nukleinsäure, welche GIRK2 codiert, umfasst.

7. Kit, umfassend drei pharmazeutische Zusammensetzungen, wobei:
- die erste pharmazeutische Zusammensetzung einen viralen Vektor umfasst, wobei der virale Vektor eine Nukleinsäure umfasst, welche RdCVF codiert,
- die zweite pharmazeutische Zusammensetzung einen viralen Vektor umfasst, wobei der virale Vektor eine Nukleinsäure umfasst, welche RdCVFL codiert, und
- die dritte pharmazeutische Zusammensetzung einen viralen Vektor umfasst, wobei der virale Vektor eine Nukleinsäure umfasst, welche GIRK2 codiert.

8. Pharmazeutische Zusammensetzung nach einem der Ansprüche 1 bis 4, viraler Vektor nach Anspruch 5 oder Kit nach Anspruch 6 oder 7, wobei das RdCVF die kurze humane Isoform hRdCVF ist, wie in SEQ ID NO:1 dargelegt.

9. Pharmazeutische Zusammensetzung nach einem der Ansprüche 1 bis 4 oder 8 oder viraler Vektor nach Anspruch 5 oder 8 oder Kit nach einem der Ansprüche 6 bis 8, wobei das RdCVFL die humane lange Isoform hRdCVFL ist, wie in SEQ ID NO:2 dargelegt.

10. Pharmazeutische Zusammensetzung nach einem der Ansprüche 1 bis 4 oder 8 bis 9 oder viraler Vektor nach einem der Ansprüche 5, 8 oder 9 oder Kit nach einem der Ansprüche 6 bis 9, wobei das GIRK2 die humane GIRK2-Sequenz ist, wie in SEQ ID NO:9 dargelegt.

11. Pharmazeutische Zusammensetzung nach einem der Ansprüche 1 bis 4 oder 8 bis 10 oder viraler Vektor nach einem der Ansprüche 5 oder 8 bis 10 oder Kit nach einem der Ansprüche 6 bis 10 zur Verwendung bei der Behandlung einer retinalen degenerativen Erkrankung.

12. Pharmazeutische Zusammensetzung nach einem der Ansprüche 1 bis 4 oder 8 bis 10 oder viraler Vektor nach einem der Ansprüche 5 oder 8 bis 10 oder Kit nach einem der Ansprüche 6 bis 10 zur Verwendung nach Anspruch 11, wobei die retinale degenerative Erkrankung eine Stäbchen-Zapfen-Dystrophie, eine Zapfen-Dystrophie, eine Zapfen-Stäbchen-Dystrophie oder eine atrophische altersbedingte Makuladegeneration ist.

13. Pharmazeutische Zusammensetzung nach Anspruch 1 bis 4 oder 8 bis 10 oder viraler Vektor nach einem der Ansprüche 5, 8 bis 10 oder Kit nach einem der Ansprüche 6 bis 10 zur Verwendung nach Anspruch 11, wobei die retinale degenerative Erkrankung ausgewählt ist aus der Gruppe bestehend aus Retinitis pigmentosa, altersbedingter Makuladegeneration, Bardet-Biedel-Syndrom, Bassen-Kornzweig-Syndrom, Morbus Best, Choroideremie, Gyrate-Atrophie, Lebersche Kongenitale Amaurose, Refsum-Krankheit, Stargardt-Krankheit oder Usher-Syndrom.

14. Pharmazeutische Zusammensetzung nach einem der Ansprüche 1 bis 4 oder 8 bis 10, viraler Vektor nach einem der Ansprüche 5 oder 8 bis 10 oder Kit nach einem der Ansprüche 6 bis 10 zur Verwendung nach Anspruch 11, wobei die retinale degenerative Erkrankung die Retinitis pigmentosa ist.

15. Pharmazeutische Zusammensetzung nach einem der Ansprüche 1 bis 4 oder 8 bis 10, viraler Vektor nach einem der Ansprüche 5 oder 8 bis 10 oder Kit nach einem der Ansprüche 6 bis 10 zur Verwendung nach einem der Ansprüche 11 bis 14, wobei die pharmazeutische Zusammensetzung oder der virale Vektor durch subretinale Injektion, intravitreale Injektion oder suprachoroidale Injektion verabreicht wird.

## Revendications

1. Composition pharmaceutique comprenant un ou plusieurs vecteurs viraux, dans laquelle lesdits un ou plusieurs vecteurs viraux comprennent un acide nucléique codant pour une isoforme courte du facteur de viabilité des cônes dérivé des bâtonnets (RdCVF), un acide nucléique codant pour une isoforme longue du facteur de viabilité des cônes dérivé des bâtonnets (RdCVFL) et un acide nucléique codant pour un canal potassique de redresseur interne 2 activé par une protéine G (GIRK2).

2. Composition pharmaceutique selon la revendication 1, dans laquelle ladite composition comprend un premier vecteur viral comprenant un acide nucléique codant pour RdCVF et un acide nucléique codant pour RdCVFL, et un deuxième vecteur viral comprenant un acide nucléique codant pour GIRK2.

3. Composition pharmaceutique selon la revendication 1, dans laquelle ladite composition comprend trois vecteurs viraux comprenant respectivement un acide nucléique codant pour RdCVF, un acide nucléique codant pour RdCVFL et un acide nucléique codant pour GIRK2.

4. Composition pharmaceutique selon la revendication 1, dans laquelle ladite composition pharmaceutique comprend un vecteur viral unique, ledit vecteur viral unique comprenant trois acides nucléiques codant respectivement pour RdCVF, RdCVFL et GIRK2.

5. Vecteur viral comprenant trois acides nucléiques codant respectivement pour RdCVF, RdCVFL et GIRK2.

6. Kit comprenant deux compositions pharmaceutiques, dans lequel :
- la première composition pharmaceutique comprend un vecteur viral qui comprend un acide nucléique codant pour RdCVF et un acide nucléique codant pour RdCVFL, et
- la deuxième composition pharmaceutique comprend un vecteur viral qui comprend un acide nucléique codant pour GIRK2.

7. Kit comprenant trois compositions pharmaceutiques, dans lequel :
- la première composition pharmaceutique comprend un vecteur viral, ledit vecteur viral comprenant un acide nucléique codant pour RdCVF,
- la deuxième composition pharmaceutique comprend un vecteur viral, ledit vecteur viral comprenant un acide nucléique codant pour RdCVFL, et
- la troisième composition pharmaceutique comprend un vecteur viral, ledit vecteur viral comprenant un acide nucléique codant pour GIRK2.

8. Composition pharmaceutique selon l'une quelconque des revendications 1 à 4, vecteur viral selon la revendication 5, ou kit selon la revendication 6 ou 7, dans lequel ledit RdCVF est l'isoforme courte humaine hRdCVF telle que présentée dans la SEQ ID NO:1.

9. Composition pharmaceutique selon l'une quelconque des revendications 1 à 4 ou 8, ou vecteur viral selon la revendication 5 ou 8, ou kit selon l'une quelconque des revendications 6 à 8, dans laquelle ledit RdCVFL est l'isoforme longue humaine hRdCVFL telle que présentée dans la SEQ ID NO:2.

10. Composition pharmaceutique selon l'une quelconque des revendications 1 à 4 ou 8 à 9, ou vecteur viral selon l'une quelconque des revendications 5, 8 ou 9, ou kit selon l'une quelconque des revendications 6 à 9, dans lequel ledit GIRK2 est la séquence GIRK2 humaine telle que présentée dans la SEQ ID NO:9.

11. Composition pharmaceutique selon l'une quelconque des revendications 1 à 4 ou 8 à 10, ou vecteur viral selon l'une quelconque des revendications 5 ou 8 à 10, ou kit selon l'une quelconque des revendications 6 à 10, pour une utilisation dans le traitement d'une maladie dégénérative rétinienne.

12. Composition pharmaceutique selon l'une quelconque des revendications 1 à 4 ou 8 à 10, ou vecteur viral selon l'une quelconque des revendications 5 ou 8 à 10, ou kit selon l'une quelconque des revendications 6 à 10, pour une utilisation selon la revendication 11, dans laquelle ladite maladie dégénérative rétinienne est une dystrophie bâtonnets-cônes, une dystrophie des cônes, une dystrophie cônes-bâtonnets ou une dégénérescence maculaire atrophique liée à l'âge.

13. Composition pharmaceutique selon les revendications 1 à 4 ou 8 à 10, ou vecteur viral selon l'une quelconque des revendications 5, 8 à 10, ou kit selon l'une quelconque des revendications 6 à 10, pour une utilisation selon la revendication 11, dans laquelle ladite maladie dégénérative rétinienne est sélectionnée dans le groupe consistant en la rétinite pigmentaire, la dégénérescence maculaire liée à l'âge, le syndrome de Bardet-Biedel, le syndrome de Bassen-Kornzweig, la maladie de Best, la choroïdémie, l'atrophie gyrée, l'amaurose congénitale de Leber, la maladie de Refsum, la maladie de Stargardt ou le syndrome d'Usher.

14. Composition pharmaceutique selon l'une quelconque des revendications 1 à 4 ou 8 à 10, vecteur viral selon l'une quelconque des revendications 5, ou 8 à 10, ou kit selon l'une quelconque des revendications 6 à 10, pour une utilisation selon la revendication 11, dans laquelle ladite maladie dégénérative rétinienne est la rétinite pigmentaire.

15. Composition pharmaceutique selon l'une quelconque des revendications 1 à 4 ou 8 à 10, vecteur viral selon l'une quelconque des revendications 5 ou 8 à 10, ou kit selon l'une quelconque des revendications 6 à 10, pour une utilisation selon l'une quelconque des revendications 11 à 14, dans laquelle ladite composition pharmaceutique ou ledit vecteur viral est administré(e) par injection sous-rétinienne, injection intravitréenne ou injection suprachoroïdienne.
